(19) Europäisches Patentamt
European Patent Office
Office européen des brevets

(11) **EP 3 895 683 A1**

(12) **DEMANDE DE BREVET EUROPEEN**

(43) Date de publication:
**20.10.2021 Bulletin 2021/42**

(51) Int Cl.:
***A61K 8/03*** *(2006.01)*   ***A61K 8/31*** *(2006.01)*
***A61K 8/92*** *(2006.01)*   ***A61Q 1/14*** *(2006.01)*
***A61Q 19/08*** *(2006.01)*

(21) Numéro de dépôt: **20315162.6**

(22) Date de dépôt: **14.04.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(71) Demandeur: **Chanel Parfums Beauté**
**92200 Neuilly-sur-Seine (FR)**

(72) Inventeurs:
• **CHICU, Irina**
**93694 Pantin Cedex (FR)**
• **FOURNIER, Odile**
**93694 Pantin Cedex (FR)**

(74) Mandataire: **Plasseraud IP**
**66, rue de la Chaussée d'Antin**
**75440 Paris Cedex 09 (FR)**

(54) **COMPOSITION COSMÉTIQUE MULTI-PHASES COMPRENANT AU MOINS DEUX PHASES HUILEUSES NON MISCIBLES**

(57) La présente invention a pour objet une composition cosmétique multi-phases comprenant deux phases huileuses non miscibles définies par des paramètres de solubilité à température ambiante spécifiques, et leur utilisation dans le domaine cosmétique ou dermatologique, et notamment pour le démaquillage, le nettoyage et/ou le soin de la peau, des yeux et/ou des phanères.

EP 3 895 683 A1

## Description

La présente invention concerne composition cosmétique multi-phases comprenant deux phases huileuses non miscibles définies par des paramètres de solubilité à température ambiante spécifiques, et leur utilisation dans le domaine cosmétique ou dermatologique, et notamment pour le démaquillage, le nettoyage et/ou le soin de la peau, des yeux et/ou des phanères.

## Domaine technique

Les compositions cosmétiques constituées de plusieurs phases distinctes, généralement d'au moins une phase aqueuse et au moins une phase huileuse, dans lesquelles lesdites phases sont séparées et non émulsionnées l'une dans l'autre au repos, sont usuellement désignées sous le terme de "compositions multi-phases". Elles se distinguent des émulsions par le fait qu'au repos, les phases sont distinctes, au lieu d'être émulsionnées l'une dans l'autre. L'utilisation de ces compositions multi-phases nécessite une agitation préalable afin de former un mélange homogène émulsionné permettant l'application simultanée de l'ensemble des ingrédients contenus dans ces phases. Au repos, lesdites phases doivent se séparer rapidement et retrouver leur état initial, ce phénomène étant plus connu sous le terme de "déphasage". L'intérêt de ce déphasage est principalement visuel : les produits obtenus sont peu courants dans le domaine et se présente sous la forme de plusieurs phases préférentiellement transparentes, dont l'aspect est ludique et attractif pour le consommateur.

Un déphasage (ou démixage) rapide des deux phases après leur utilisation constitue une des qualités recherchées des compositions biphasés. En effet, l'obtention d'un déphasage rapide est souhaitable pour diverses raisons, notamment parce qu'une mauvaise séparation des deux phases est perçue comme étant inesthétique par les utilisateurs.

Les compositions multi-phases classiques sont généralement des compositions biphases constituées d'une phase aqueuse et d'une phase huileuse. De telles compositions comprenant une seule phase huileuse ne sont pas optimales en termes de propriétés sensorielles. Si un effet de fraicheur est généralement obtenu par la présence de la phase aqueuse, ces compositions manquent souvent de confort, et peuvent tirailler. En outre, l'ajout d'actifs cosmétiques est conditionné par la polarité des phases mises en œuvre. Dans des compositions biphases classiques, on peut donc introduire des actifs solubles en phase polaire et des actifs solubles dans la phase huileuse sélectionnée, mais en l'absence de phase intermédiaire, toute une catégorie d'ingrédients actifs sont exclus.

En outre, avec les compositions biphases classiques, une émulsion n'est pas nécessairement formée après agitation de la composition, ce qui aboutit à un démaquillage non satisfaisant, et laisse un résidu gras sur la peau ou la matière kératinique.

On a donc, par le passé, cherché à formuler des compositions biphases constituées de deux phases distinctes non miscibles, qui, après agitation et formation extemporanée et éphémère de l'émulsion, présentent un déphasage rapide en deux phases, sans formation de mousse. De telles compositions biphases formant en outre, après agitation préalable, une émulsion éphémère permettant l'application de la composition et un démaquillage efficace, et ce, sans formation de résidu gras sur la peau ou la matière kératinique.

Une composition de ce type a par exemple été décrite dans la demande FR-A-3049459 par la mise en œuvre d'un système émulsionnant très spécifique, comprenant un dérivé d'inuline, un lysophospholipide, un poloxamère, un polypropylène glycol buteth et un dérivé d'huile de ricin hydrogénée.

Toutefois, ce document n'enseigne pas comment opérer une composition multi-phases, en particulier tri-phases, comprenant au moins deux phases huileuses non miscibles. En outre, la mise en œuvre de tensioactifs est susceptible de poser des problèmes d'intolérance, en particulier chez les sujets présentant une peau sensible, ce qu'il est souhaitable d'éviter pour les produits cosmétiques tels que les produits de soin.

Enfin, on souhaite de plus en plus limiter l'utilisation de matières premières d'origine synthétique telles que les silicones, les dérivés fluorés, les polyéthylène ou polypropylène-glycols, du fait de l'impact négatif de leur synthèse sur l'environnement. Or ces matières premières sont bien souvent essentielles pour procurer les propriétés sensorielles de confort et de douceur, ou pour permettre la séparation des phases dans les produits multi-phases.

On demeure donc à la recherche d'une formulation multi-phases comprenant au moins deux phases huileuses non miscibles, présentant une séparation de phases très nette sans nécessiter la mise en œuvre de tensioactifs, et conférant des bonnes propriétés sensorielles en termes de douceur, confort, et tolérance.

La présente invention a ainsi pour objet une composition cosmétique multi-phases comprenant :

- une première phase huileuse polaire caractérisée par une tension de surface supérieure à 30 mN/m, et présentant des paramètres de solubilité à température ambiante $\delta_p$ compris entre 6 et 7,8 et $\delta_h$ compris entre 3,7 et 10,1
- une seconde phase huileuse apolaire, non miscible avec la première phase huileuse, caractérisée par une tension de surface inférieure à 28 mN/m, et présentant des paramètres de solubilité à température ambiante $\delta_p$ et $\delta_h$ différents de ceux du solvant polaire mis en œuvre dans la première phase huileuse, ladite composition étant exempte de tensioactif.

Par « exempte de tensioactif », on entend, au sens de la présente demande, que la composition comprend moins de 2% en poids, de préférence moins de 1% en poids, plus préférentiellement moins de 0,5% en poids, de tensioactifs.

La titulaire s'est en effet aperçue que, outre les problèmes d'intolérance identifiés liés à l'utilisation de tensioactifs dans des produits multi-phases, certains tensioactifs tels que les aklylpolyglucosides conféraient à la composition un effet blanchissant non souhaité lors de son application, dû au phénomène de foisonnement (dit « savonnage »). Le système mis au point dans la présente invention permet donc en outre d'éviter le blanchiment à l'application.

La composition cosmétique selon l'invention est constituée d'au moins deux phases huileuses distinctes, s'émulsionne facilement par agitation et se déphase facilement après arrêt de l'agitation.

La composition selon l'invention est de préférence liquide et destinée à une application topique.

L'invention a également pour objet un produit de soin de la peau, comprenant une telle composition, un procédé de soin de la peau, consistant à appliquer sur la peau une telle composition et l'utilisation d'une telle composition dans le domaine cosmétique ou dermatologique, et notamment pour le démaquillage, le nettoyage et/ou le soin de la peau, des yeux et/ou des phanères.

Première phase huileuse

La composition selon l'invention met en œuvre une première phase huileuse polaire caractérisée par une tension de surface supérieure à 30 mN/m, de préférence supérieure à 35 mN/m, plus préférentiellement supérieure à 40 mN/m et présentant des paramètres de solubilité à température ambiante $\delta_p$ compris entre 6 et 7,8 et $\delta_h$ compris entre 3,7 et 10,1.

Par « température ambiante », on entend une température d'environ 25 °C.

En particulier, la première phase huileuse polaire comprend un ou plusieurs solvants polaires.

Les solvants polaires mis en œuvre dans la première phase huileuse peuvent être caractérisés par cinq paramètres de solution : quatre paramètres de solubilité d'Hansen ($\delta_d$, $\delta_p$, $\delta_h$ et $\delta_T$) et Log ($K_{ow}$).

Les paramètres de solubilité d'Hansen ($\delta_d$, $\delta_p$, $\delta_h$ et $\delta_T$) permettent de prédire si une substance sera solubilisée dans une autre.

$\delta_d$ est une contribution due aux forces de dispersion, $\delta_p$ est une contribution due aux forces polaires, $\delta_h$ est une contribution de lien hydrogène. Ces paramètres sont définis comme suit :

$$\delta_d = \left(\frac{\Delta E_d}{V}\right)^{1/2}$$

$$\delta_p = \left(\frac{\Delta E_p}{V}\right)^{1/2}$$

$$\delta_h = \left(\frac{\Delta E_h}{V}\right)^{1/2}$$

Ici, $\Delta E$ correspond à l'énergie de vaporisation et V le volume molaire du liquide.

$\delta_T$ est le paramètre de solubilité dû à la contribution de chacun de ces paramètres :

$$\delta_T = \left(\delta_d^2 + \delta_p^2 + \delta_h^2\right)^{1/2}$$

Log ($K_{ow}$) est une mesure de la solubilité différentielle d'un composé chimique dans deux solvants (octanol et eau) et correspond au coefficient de partage octanol/eau.

La Demanderesse a maintenant découvert qu'il était possible de réaliser une composition multi-phases comprenant au moins deux phases huileuses en mettant en œuvre une première phase huileuse polaire présentant des paramètres de solubilité à température ambiante $\delta_p$ compris entre 6 et 7,8 et $\delta_h$ compris entre 3,7 et 10,1, et une seconde phase huileuse présentant des paramètres de solubilité à température ambiante $\delta_p$ et $\delta_h$ différents de la première phase huileuse.

Dans un mode de réalisation particulier, le ou les solvants polaires de la première phase huileuse selon la présente invention présentent une valeur de $\delta_p$ comprise entre 6 et 7,8 à température ambiante.

Dans un mode de réalisation particulier, le ou les solvants polaires de la première phase huileuse selon la présente invention présentent une valeur de $\delta_h$ compris entre 3,7 et 10,1 à température ambiante.

Dans un mode de réalisation particulier, le ou les solvants polaires de la première phase huileuse présentent un paramètre de solubilité à température ambiante $\delta_d$ compris entre 16 et 19.

Les valeurs des différents paramètres de solubilité des solvants peuvent par exemples être calculées par le logiciel HSPiP (par exemple disponible sur le site https://www.hansen-solubility.com/buy-HSPiP-software.php). Ce logiciel calcule les paramètres de solubilité d'Hansen ($\delta_d$, $\delta_p$, $\delta_h$ et $\delta_T$) de chaque solvant en se basant sur la composition dudit solvant en acides gras en C8 à C18. Sur la base des valeurs connues des paramètres de solubilité d'Hansen ($\delta_d$, $\delta_p$, $\delta_h$ et $\delta_T$) reprises dans le tableau 1, le logiciel peut calculer les paramètres propres au solvant en fonction de la proportion massique de chaque acide gras dans le solvant considéré :

[Tableau 1]

| Nom INCI | $\delta d$ | $\delta p$ | $\delta h$ |
|---|---|---|---|
| Acide Caprylique C8:0 | 16.3 | 5.2 | 10.1 |
| Acide Caprique C10:0 | 16.3 | 4.2 | 8.6 |
| Acide Laurique C12:0 | 16.3 | 4 | 7.5 |
| Acide Myristique C14:0 | 16.3 | 3.3 | 6.6 |
| Acide Palmitique C16:0 | 16.2 | 3.3 | 5.8 |
| Acide Margarique C17:0 | 16.2 | 2.8 | 5.4 |
| Acide Stearique C18:0 | 16.2 | 2.8 | 5.2 |
| Acide Arachidique C20:0 | 16.1 | 2.7 | 4.7 |
| Acide Béhénique C22:0 | 16.2 | 2.3 | 4.3 |
| Acide Lignocerique C24:0 | 16.1 | 2.4 | 3.9 |
| Acide palmitoleique C16:1 | 16.5 | 3.4 | 6.3 |
| Acide Oléique C18:1 | 16.4 | 3 | 5.5 |
| Acide Gadoléique C20:1 | 16.4 | 2.9 | 5.1 |
| Acide Erucique C22:1 | 16.3 | 2.6 | 4.5 |
| Acide Linoléique C18:2 | 16.7 | 3.1 | 6.1 |
| Acide Linolénique C18:3 | 16.9 | 3.3 | 5.8 |
| Ricinoleic acid | 16.5 | 4.4 | 8.2 |

Le logiciel HSPiP base par exemple son calcul sur la méthode Stefanis-Panayiotou par exemple décrite dans l'articl Stefanis, E., Panayiotou, C. Prediction of Hansen Solubility Parameters with a New Group-Contribution Method. Int J Thermophys 29, 568-585 (2008). La méthode de Hoy et Van Krevelen ou la méthode automatisée Y-MB (Yamamoto-Molecule Breaking) peuvent également être considérées.

Lorsque la première phase huileuse comprend un mélange de solvants, les paramètres de solubilité sont calculés en fonction de la proportion massique de chaque acide gras dans le milieu. En outre, le ou les solvants polaires de la première phase huileuse présentent une tension de surface supérieure à 30 mN/m, de préférence encore supérieure à 35 mN/m, et plus préférentiellement à supérieure à 40 mN/m.

La tension de surface peut quant à elle être prédite par le logiciel HSPiP.

Dans un mode de réalisation particulier, le ou les solvants polaires de la première phase huileuse ont une valeur de $\log(K_{ow})$ comprise entre 0,86 et 4,37 à température ambiante.

Dans un mode de réalisation particulier, le ou les solvants polaires de la première phase huileuse sont choisis parmi le dibenzoate de dipropylène glycol, le dibenzoate de propylène glycol, le citrate de triéthyle, l'huile de ricin, ou un mélange de ceux-ci. Ces solvants polaires présentent les paramètres de solubilité présentés dans le tableau 2 suivant :

[Tableau 2]

| Nom INCI | $\delta d$ | $\delta p$ | $\delta h$ | Tension de surface (mN/m) |
|---|---|---|---|---|
| Dipropylene Glycol Dibenzoate | 18 | 6,2 | 3,7 | 51,60 |
| Propylene Glycol Dibenzoate | 18,4 | 6,5 | 3,7 | 52,50 |
| Triethyl citrate | 16,8 | 6,0 | 10,1 | 42,00 |
| Huile de ricin | 18,2 | 7,8 | 9,3 | 44,49 |

Le solvant polaire de la première phase huileuse peut représenter de 80% à 100 % en poids, et de préférence de 95% à 99,9 % en poids, de ladite première phase huileuse.

Le solvant polaire de la première phase huileuse peut être présent, dans la composition de la présente invention, en une proportion allant de 5 à 50 % en poids, et de préférence de 8 % à 25 % en poids, par rapport au poids total de la composition.

Outre le ou les solvants polaires, il est possible d'ajouter, à la première phase huileuse, une huile moyennement polaire telle que l'huile d'argan, l'huile de graine de limnanthe, l'huile de jojoba, ou un mélange de celles-ci sous réserve que ladite phase huileuse présente toujours une tension de surface supérieure à 30 mN/m, et des paramètres de solubilité à température ambiante $\delta_p$ compris entre 6 et 7,8 et $\delta_h$ compris entre 3,7 et 10,1.

Ces huiles moyennement polaires présentent les paramètres de solubilité présentés dans le tableau 3 suivant :

[Tableau 3]

| Nom INCI | $\delta d$ | $\delta p$ | $\delta h$ | Tension de surface (mN/m) |
|---|---|---|---|---|
| Huile d'argan | 16,5 | 3,0 | 5,7 | 33,50 |
| Huile de graine de limnanthe | 16,4 | 2,8 | 5,0 | 35,20 |
| Huile de jojoba | 16,6 | 3,1 | 5,3 | 35,33 |

Deuxième phase huileuse

La composition selon l'invention met en œuvre une seconde phase huileuse, non miscible avec la première phase huileuse, caractérisée par une tension de surface inférieure à 28 mN/m, et présentant des paramètres de solubilité à température ambiante $\delta_p$ et $\delta_h$ différents de ceux de la première phase huileuse. En particulier, la deuxième phase huileuse présente des paramètres de solubilité à température ambiante $\delta_p$ comprise entre 0 et 1, $\delta_h$ comprise entre 0 et 1 et/ou $\delta_d$ compris entre 15,6 et 15,9.

En particulier, la deuxième phase huileuse apolaire comprend un ou plusieurs solvants apolaires.

Dans un mode de réalisation particulier, le ou les solvants apolaires de la deuxième phase huileuse selon la présente invention présentent une valeur de $\delta_p$ comprise entre 0 et 1 à température ambiante, de préférence entre 0,01 et 0,5, et plus préférentiellement de 0,1.

Dans un mode de réalisation particulier, le ou les solvants apolaires de la deuxième phase huileuse selon la présente invention présentent une valeur de $\delta_h$ comprise entre 0 et 1 à température ambiante, de préférence entre 0,01 et 0,5, et plus préférentiellement de 0,1.

Dans un mode de réalisation particulier, le ou les solvants apolaires de la deuxième phase huileuse présentent un paramètre de solubilité à température ambiante $\delta_d$ compris entre 15,6 et 15,9.

En outre, le ou les solvants apolaires de la deuxième phase huileuse présentent une tension de surface inférieure à 28 mN/m.

Dans un mode de réalisation particulier, le ou les solvants apolaires de la deuxième phase huileuse présentent une valeur de log($K_{ow}$) comprise entre 6,3 et 27,7 à température ambiante.

Dans un mode de réalisation particulier, le ou les solvants apolaires de la deuxième phase huileuse sont choisis parmi les hydrocarbures, les polymères obtenus à partir d'un monomère hydrocarbure, ou un mélange de ceux-ci.

Les hydrocarbures ou les polymères obtenus à partir d'un monomère hydrocarbure sont, de préférence, choisis parmi l'isohexadécane, l'isododécane, l'undécane, le tridécane, le polyisobutène, le squalane ou un mélange de ceux-ci.

Ces solvants apolaires présentent les paramètres de solubilité présentés dans le tableau 4 suivant :

[Tableau 4]

| Nom INCI | δd | δp | δh | Tension de surface (mN/m) |
|---|---|---|---|---|
| UNDECANE | 15.7 | 0.1 | 0.1 | 23,6 |
| TRIDECANE | 15.6 | 0.1 | 0.1 | 25,7 |
| CETIOL ULTIMATE (Undécane+Tridécane) | 15.6 | 0.1 | 0.1 | 23,75 |
| SQUALANE | 15.9 | 0.1 | 0.1 | 27,65 |
| ISOHEXADECANE | 15.7 | 0.1 | 0.1 | 27,1 |
| ISODODECANE | 15.6 | 0.1 | 0.1 | 24,3 |

Le ou les solvants apolaires de la deuxième phase huileuse peuvent représenter de 80% à 100 % en poids, et de préférence de 95% à 99,9 % en poids, de ladite première phase huileuse.

Le ou les solvants apolaires de la deuxième phase huileuse peuvent être présents, dans la composition de la présente invention, en une proportion allant de 1 à 50 % en poids, et de préférence de 15 % à 25 % en poids, par rapport au poids total de la composition.

Outre le ou les solvants apolaires, il est possible d'ajouter, à la deuxième phase huileuse, des huiles naturelles de polarités moyennes décrites précédemment, telles que l'huile d'argan, l'huile de graine de limnanthe, l'huile de jojoba, ou un mélange de celles-ci sous réserve que ladite phase huileuse présente toujours une tension de surface inférieure à 28 mN/m, et des paramètres de solubilité à température ambiante $\delta_p$ et $\delta_h$ différents de ceux de la première phase huileuse.

Dans un mode préféré de réalisation, les paramètres de solubilité à température ambiante des solvants polaire et apolaire mis en œuvre respectivement dans les première et deuxième phases huileuses sont tels que

$$\sqrt{(\delta dpolaire - \delta dapolaire)^2 + (\delta ppolaire - \delta papolaire)^2 + (\delta hpolaire - \delta h2apolaire)^2}$$

est supérieur à 3,5.

Cette caractéristique permet de garantir l'immiscibilité des deux phases huileuses. De préférence, les paramètres de solubilité à température ambiante des solvants polaire et apolaire mis en œuvre respectivement dans les première et deuxième phases huileuses sont tels que

$$\sqrt{(\delta dpolaire - \delta dapolaire)^2 + (\delta ppolaire - \delta papolaire)^2 + (\delta hpolaire - \delta h2apolaire)^2}$$

est supérieur à 3,8.

Phase aqueuse

Selon un mode préféré de réalisation, la composition selon l'invention comprend une troisième phase aqueuse, non miscible avec les premières et deuxième phases huileuses.

La phase aqueuse de la composition selon l'invention comprend de l'eau et tout additif hydrosoluble ou hydrodispersible.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle, comme par exemple: l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène.

Comme additifs hydrosolubles, on peut citer notamment les polyols tels que le propylène glycol, le butylène glycol, le pentanediol, l'hexylèneglycol, les polyéthylène glycols, le néopentyl glycol, le glycérol. Les polyols peuvent être présents en une quantité allant de 0,01 à 10% en poids, de préférence de 0,05 à 8% en poids par rapport au poids total de la composition. Selon un mode préféré de réalisation de l'invention, la composition comprend au moins un polyol, de préférence la glycérine (le glycérol).

L'additif hydrosoluble peut également être un sel, tel que par exemple le chlorure de sodium, qui est utilisé comme adjuvant dans une composition de démaquillage des yeux pour que la composition ait une pression osmotique proche de celle des larmes. La présence de sel permet l'obtention d'une phase aqueuse limpide. La quantité de sel(s) peut aller par exemple de 0,01 à 3% en poids et de préférence de 0,05 à 2% en poids et mieux de 0,1 à 1% en poids par rapport au poids total de la composition.

Actif cosmétique

La composition selon l'invention peut également contenir des adjuvants ou additifs cosmétiques conventionnels qui se trouveront dans l'une ou l'autre phase selon leur nature hydrophile ou lipophile, polaire ou apolaire.

Les actifs cosmétiques peuvent être choisis parmi les vitamines, les antioxydants, les agents hydratants, les agents anti-pollution, les agents kératolytiques, des astringents, des anti-inflammatoires, des agents blanchissants, les agents favorisant la microcirculation, les colorants, les agents adoucissants, les tampons, les filtres UV (ou filtres solaires), les parfums, les ajusteurs de pH (par exemple acide citrique ou hydroxyde de sodium), et leurs mélanges.

Des exemples de vitamines incluent les vitamines A, B1, B2, B6, C et E et leurs dérivés, l'acide pantothénique et ses dérivés et la biotine.

Des exemples d'antioxydants incluent l'acide ascorbique et ses dérivés tels que le palmitate d'ascorbyle, le tétraisopalmitate d'ascorbyle, l'ascorbyl glucoside, le magnésium ascorbyl phosphate, le sodium ascorbyl phosphate et le sorbate d'ascorbyle; le tocophérol et ses dérivés, tels que l'acétate de tocophérol, le sorbate de tocophérol et d'autres esters de tocophérol; le BHT et BHA; les esters de l'acide gallique, l'acide phosphorique, l'acide citrique, l'acide maléique, l'acide malonique, l'acide succinique, l'acide fumarique, la céphaline, l'hexamétaphosphate, l'acide phytique, et les extraits de plantes, par exemple de racines de Zingiber Officinale (Gingembre) tel que le Blue Malagasy Ginger commercialisé par la société BIOLANDES, de Chondrus crispus, Rhodiola, Thermus thermophilus, la feuille de maté, le bois de chêne, l'écorce de Rapet Kayu, les feuilles de Sakura et les feuilles d'ylang ylang.

Des exemples d'agents hydratants incluent le polyéthylène glycol, le propylène glycol, le dipropylène glycol, la glycérine, le butylène glycol, le xylitol, le sorbitol, le maltitol, les mucopolysaccharides, tels que l'acide chondroïtine sulfurique, l'acide hyaluronique de haut ou de bas poids moléculaire ou encore l'acide hyaluronique potentialisé par un dérivé de silanol tel que l'actif Epidermosil® commercialisé par la société Exymol, et l'acide mucoitinsulfurique; l'acide caronique; l'atelo collagène; le chlorestéryl-12-hydroxystéarate; les sels biliaires, une composante principale du FHN (facteur d'hydratation naturelle) comme un sel de l'acide pyrrolidone carboxylique et un sel d'acide lactique, un analogue d'acide aminé tel que l'urée, la cystéine et la sérine; un collagène soluble à chaîne courte, les PPG diglycérine, les homo- et copolymères de 2-méthacryloyloxyéthylphosphorylcholine comme le Lipidure HM et le Lipidure PBM de NOF; l'allantoïne; des dérivés de glycérine tels que le PEG / PPG / polybutylène Glycol-8/5/3 Glycérine de NOF vendu sous la dénomination commerciale Wilbride®S753 ou encore le glyceryl-polymethacrylate de Sederma vendu sous la dénomination commerciale Lubragel®MS; la triméthylglycine vendu sous la dénomination commerciale Aminocoat® par la société Ashahi Kasei Chemicals et divers extraits de plantes tels que des extraits de Castanea sativa, des protéines de noisette hydrolysées, les polysaccharides de Tuberosa Polyanthes, l'huile de noyau d'Argania spinosa et les extraits de nacre contenant un conchyoline qui sont vendus notamment par la compagnie Maruzen (Japon) sous le nom commercial Pearl Extract®.

D'autres exemples d'agents hydratants incluent les composés stimulant l'expression de la matriptase MT/SP1, tel qu'un extrait de pulpe de caroube, ainsi que les agents stimulant l'expression de CERT, d'ARNT2 ou de FN3K ou FN3K RP ; les agents augmentant la prolifération ou la différenciation des kératinocytes, soit directement, soit indirectement en stimulant par exemple la production de β-endorphines, tels que les extraits de *Thermus thermophilus* ou de coques de fèves de *Theobroma cacao,* les extraits hydrosolubles de maïs, les extraits peptidiques de *Voandzeia subterranea* et le niacinamide ; les lipides épidermiques et les agents augmentant la synthèse de lipides épidermiques, soit directement, soit en stimulant certaines β-glucosidases qui modulent la déglycosylation de précurseurs lipidiques comme le glucosylcéramide en céramides, tels que les phospholipides, les céramides, les hydrolysats de protéine de lupin et les dérivés d'acide dihydrojasmonique.

Des exemples d'agents anti-pollution incluent l'extrait de graines de Moringa pterygosperma (par exemple le Purisoft® de LSN); l'extrait de beurre de karité (par exemple Detoxyl® de Silab), un mélange d'extrait de lierre, d'acide phytique, d'extrait de graine de tournesol (par exemple l'Osmopur® de Sederma).

Des exemples d'agents kératolytiques incluent les α-hydroxyacides (par exemple les acides glycolique, lactique, citrique, malique, mandélique, ou tartrique) et les β-hydroxyacides (par exemple l'acide salicylique), et leurs esters, tels que les C12-13 alkyl lactates, et les extraits de plantes contenant ces hydroxyacides, tels que des extraits d'Hibiscus sabdriffa.

Des exemples d'astringents incluent les extraits d'hamamélis.

Des exemples d'agents anti-inflammatoires incluent le bisabolol, l'allantoïne, l'acide tranexamique, l'oxyde de zinc, l'oxyde de soufre et ses dérivés, le sulfate de chondroïtine, l'acide glycyrrhizinique et ses dérivés tels que les glycyrrhizinates.

Des exemples d'agents blanchissants incluent l'arbutine et ses dérivés, l'acide férulique (tel que le Cytovector® : eau, glycol, lécithine, acide férulique, hydroxyéthylcellulose, commercialisé par BASF) et ses dérivés, l'acide kojique, le résorcinol, l'acide lipoïque et ses dérivés tel que le monolipoate de resvératrol diacétate tel que décrit dans la demande de brevet WO2006134282, l'acide ellagique, le leucodopachrome et ses dérivés, la vitamine B3, l'acide linoléique et ses dérivés, les céramides et leurs homologues, un peptide tel que décrit dans la demande de brevet WO2009010356, un bioprécurseur tel que décrit dans la demande de brevet WO2006134282 ou un sel de tranexamate tel que le sel de chlorhydrate de tranexamate cétylique, un extrait de réglisse (extrait de Glycyrrhiza glabra), qui est vendu notamment par la société Maruzen sous le nom commercial Licorice extract®, un agent blanchissant ayant également un effet antioxydant, comme les composés de vitamine C, y compris les sels d'ascorbate, les esters ascorbyle d'acides gras ou d'acide sorbique, et d'autres dérivés de l'acide ascorbique, par exemple, les phosphates d'ascorbyle, tels que le magnésium ascorbyl phosphate et le sodium ascorbyl phosphate, ou les esters de saccharide d'acide ascorbique, qui incluent, par exemple, l'ascorbyle-2-glucoside, le L-ascorbate de 2-O-alpha-D-glucopyranosyle, ou le L-ascorbate de 6-O-bêta-D-galactopyranosyle. Un agent actif de ce type est vendu en particulier par la société DKSH sous le nom commercial Ascorbyl glucoside®.

Des exemples d'agents favorisant la microcirculation incluent un extrait de lupin (tel que l'Eclaline® de Silab), de ruscus, de marron d'inde, de lierre, de ginseng ou de mélilot, la caféine, le nicotinate et ses dérivés, un extrait d'algue de Corallina officinalis tel que celui commercialisé par CODIF ; et leurs mélanges. Ces agents actifs sur la microcirculation cutanée peuvent être utilisés pour éviter le ternissement du teint et/ou améliorer l'homogénéisation et l'éclat du teint.

Parmi les agents adoucissants, on peut en particulier citer l'allantoïne, le bisabolol, les planctons, et certains extraits de plantes comme les extraits de rose et les extraits de mélilot.

Selon un mode préféré de réalisation, l'actif cosmétique est un actif hydratant. Selon un mode encore plus préféré de réalisation, l'actif hydratant est l'acide hyaluronique.

La composition selon l'invention peut comprendre de 0,0001 à 10% en poids d'actif cosmétique, de préférence de 0,001 à 5% en poids, et plus préférentiellement de 0,002 à 1% en poids, par rapport au poids total de la phase aqueuse.

De préférence, la composition selon l'invention présente un pH compris entre 3,5 et 7,5.

Les compositions selon l'invention sont de préférence exemptes de silicones, dérivés fluorés, polyéthylène ou polypropylène-glycols. On entend par exempte de silicone, dérivés fluorés, polyéthylène ou polypropylène-glycols que la composition comprend moins de 1% en poids de chacun de ces composés, de préférence moins de 0,5%.

En outre, les compositions selon l'invention sont exemptes de mono-alcools, c'est-à-dire qu'elle en comprend moins de 3% en poids, de préférence moins de 2% en poids, de préférence encore moins de 1% en poids, et plus préférentiellement moins de 0,5% en poids. Les alcools sont usuellement utilisés dans la phase aqueuse pour apporter un effet de frais aux compositions cosmétiques. Ils présentent toutefois un effet desséchant pour la peau et une mauvaise tolérance pour les peaux sensibles. Les inventeurs ont observé qu'il était possible de proposer, au moyen des compositions selon l'invention, des compositions sans alcool présentant un effet de frais.

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les différentes phases de la composition soient introduites dans deux compartiments indépendants d'un même flacon, un système étant prévu pour leur mélange au moment de la distribution.

La composition selon l'invention peut être utilisée pour toute application topique ; notamment, elle peut constituer une composition cosmétique ou dermatologique. Elle peut en particulier être utilisée pour le soin, le nettoyage et/ou le démaquillage de la peau, des yeux et/ou des phanères.

La présente invention a également pour objet un produit de soin de la peau, caractérisé en ce qu'il comprend une composition telle que définie précédemment.

Selon un mode préféré de réalisation de l'invention, ledit produit peut être un sérum anti-âge, de préférence présentant un effet exfoliant, ou un démaquillant pour le visage, en particulier les yeux et/ou les lèvres.

La présente invention a aussi pour objet un procédé de soin de la peau, consistant à appliquer sur la peau une composition telle que définie précédemment. En particulier, il peut s'agir d'un procédé cosmétique de démaquillage, de nettoyage et/ou de soin de la peau, des yeux et/ou des phanères, comprenant l'application sur la peau, les yeux et/ou les phanères, d'une composition cosmétique telle que définie ci-dessus.

La présente invention a aussi pour objet un procédé de préparation d'une composition telle que définie ci-dessus, comprenant les étapes suivantes :

- la préparation à froid de chacune des phases par mélange des ingrédients à la défloculeuse,
- introduction de la seconde phase huileuse apolaire dans la première phase huileuse polaire.

Lorsque la composition comprend une phase aqueuse, le procédé met en œuvre l'introduction du mélange des phases huileuses apolaire et polaire dans la phase aqueuse.

**Brève description des dessins**

**Fig. 1**

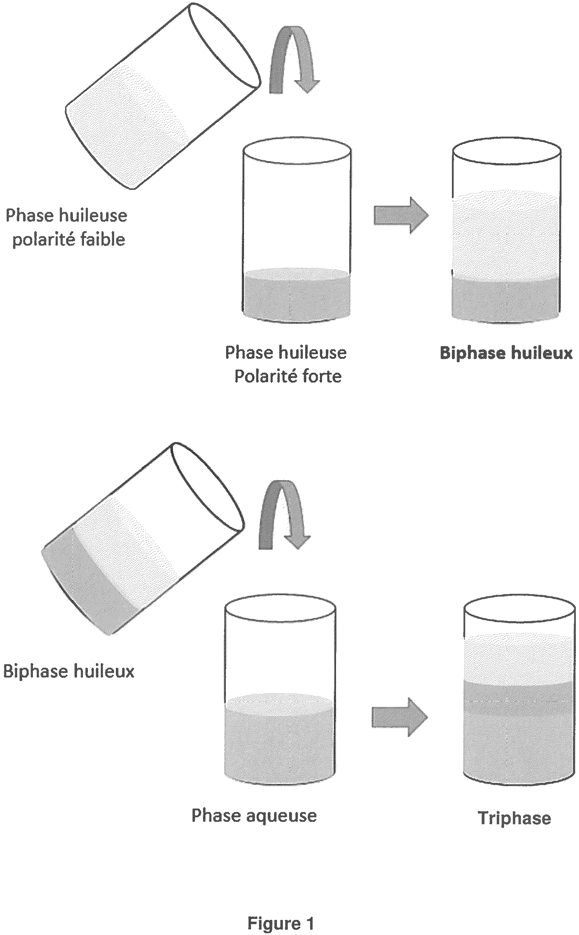

[Fig. 1] illustre le procédé de préparation d'une composition selon l'invention.

Les exemples ci-après de compositions selon l'invention est donné à titre d'illustration et sans caractère limitatif. Les quantités y sont données en % en poids, sauf mention contraire.

**Exemples**

Exemple 1 : Sérum anti-âge

On a préparé une composition triphase ayant la composition suivante.

[Tableau 5]

| | Nom INCI | Teneur (% en poids) |
|---|---|---|
| Phase aqueuse | Eau déminéralisée | 41,860% |
| | NATURAL AHA COMPLEX | 10,000% |
| | Digluconate de chlorhexidine | 0,22% |
| | Actif hydrosoluble | 4,04% |
| | Glycérine | 3,00% |
| | Butylène glycol | 4,000% |
| | Colorant | 0,03% |
| | L-ARGININE | 2,75% |
| Première phase huileuse | Huile de ricin | 15,00% |
| | Parfum | 0,10% |
| Deuxième | Squalane | 9,00% |
| phase huileuse | CETIOL ULTIMATE (undecane & tridecane) | 10,00% |

On a préparé chaque phase séparément, à froid par mélange des ingrédients de chaque phase au moyen d'un barreau aimanté. On a ensuite introduit la deuxième phase huileuse dans la première phase huileuse, puis on a introduit le mélange dans la phase aqueuse.

Le sérum ainsi obtenu se présente sous la forme d'une composition présentant visuellement trois phases non miscibles bien distinctes. Il confère un effet exfoliant/peeling lors de son application sur la peau.

Exemple 2 : Démaquillant visage

On a préparé une composition triphase ayant la composition suivante.

[Tableau 6]

| | Nom INCI | Teneur (% en poids) |
|---|---|---|
| Phase aqueuse | Eau déminéralisée | 64,578% |
| | Digluconate de chlorhexidine | 0,220% |
| | Glycérine | 4,000% |
| | P extract G (actif) | 0,200% |
| | L-ARGININE | 0,002% |
| Première phase huileuse | Huile de ricin | 10,000% |
| | Actif | 0,100% |

(suite)

|  | Nom INCI | Teneur (% en poids) |
|---|---|---|
| Deuxième phase huileuse | Cetiol Ultimate (undécance et tridécane) | 6,000% |
|  | Squalane | 9,000% |
| Huiles additionnelles | Limnanthes Alba Seed Oil | 7,000% |
|  | Huile de jojoba décolorée | 10,000% |

On a préparé chaque phase séparément, à froid par mélange des ingrédients de chaque phase au moyen d'un barreau aimanté. On a ensuite introduit la deuxième phase huileuse dans la première phase huileuse, puis on a introduit le mélange dans la phase aqueuse.

Le démaquillant visage ainsi obtenu se présente sous la forme d'une composition présentant visuellement trois phases non miscibles bien distinctes. Il démaquille en douceur la peau, les yeux et les lèvres.

**Revendications**

1.   Composition cosmétique multi-phase comprenant :

   - une première phase huileuse polaire **caractérisée par** une tension de surface supérieure à 30 mN/m, et présentant des paramètres de solubilité à température ambiante $\delta_p$ compris entre 6 et 7,8 et $\delta_h$ compris entre 3,7 et 10,1
   - une seconde phase huileuse apolaire, non miscible avec la première phase huileuse, **caractérisée par** une tension de surface inférieure à 28 mN/m, et présentant des paramètres de solubilité à température ambiante $\delta_p$ et $\delta_h$ différents de ceux du solvant polaire mis en œuvre dans la première phase huileuse, ladite composition étant exempte de tensioactif.

2.   Composition selon la revendication 1, dans laquelle le ou les solvants polaires mis en œuvre dans la première phase huileuse présente un paramètre de solubilité à température ambiante $\delta_d$ compris entre 16 et 19.

3.   Composition selon l'une quelconque des revendications précédentes, dans laquelle le ou les solvants apolaires mis en œuvre dans la deuxième phase huileuse les présentent les paramètres de solubilité à température ambiante suivants :

   - $\delta_p$ entre 0 et 1, de préférence entre 0,01 et 0,5, et plus préférentiellement de 0,1
   - $\delta_h$ entre 0 et 1, de préférence entre 0,01 et 0,5, et plus préférentiellement de 0,1 et
   - $\delta_d$ entre 15,6 et 15.9.

4.   Composition selon l'une quelconque des revendications précédentes, dans laquelle les paramètres de solubilité à température ambiante des solvants polaires et apolaires sont tels que

$$\sqrt{(\delta dpolaire - \delta dapolaire)^2 + (\delta ppolaire - \delta papolaire)^2 + (\delta hpolaire - \delta h2apolaire)^2}$$

est supérieur à 3,5.

5.   Composition selon l'une quelconque des revendications précédentes, dans laquelle le ou les solvants polaires de la première phase huileuse sont choisis parmi le dibenzoate de dipropylène glycol, le dibenzoate de propylène glycol, le citrate de triéthyle, l'huile de ricin, ou un mélange de ceux-ci.

6.   Composition selon l'une quelconque des revendications précédentes, dans laquelle le ou les solvants apolaires de la deuxième phase huileuse sont choisis parmi les hydrocarbures,les polymères obtenus à partir d'un monomère hydrocarbure, ou un mélange de ceux-ci.

7.   Composition selon la revendication 6, dans laquelle les hydrocarbures ou les polymères obtenus à partir d'un monomère hydrocarbure sont choisis parmi l'isohexadécane, l'isododécane, l'undécane, le tridécane, le polyisobutène, le squalane ou un mélange de ceux-ci.

8. Composition selon l'une quelconque des revendications précédentes, comprenant des huiles naturelles de polarités moyennes telles que l'huile d'argan, l'huile de graine de limnanthe, l'huile de jojoba, ou un mélange de celles-ci sous réserve de ne pas modifier les paramètres de solubilité à température ambiante $\delta_p$ et $\delta_h$ des première et seconde phases huileuses.

9. Composition selon l'une quelconque des revendications précédentes, comprenant une troisième phase aqueuse, non miscible avec les première et deuxième phases huileuses.

10. Composition selon la revendication 7, dans laquelle la phase aqueuse comprend de l'eau, et optionnellement au moins un polyol et/ou au moins un sel.

11. Composition selon la revendication 8, dans laquelle, le polyol est choisi parmi le propylène glycol, le butylène glycol, le pentanediol, l'hexylèneglycol, les polyéthylène glycols, le néopentyl glycol, le glycérol, et de préférence, le polyol est le glycérol.

12. Composition selon l'une quelconque des revendications précédentes, comprenant un actif cosmétique, de préférence choisi parmi les vitamines, les antioxydants, les agents hydratants, les agents anti-pollution, les agents kératolytiques, des astringents, des anti-inflammatoires, des agents blanchissants, les agents favorisant la microcirculation, les colorants, les agents adoucissants, les tampons, les filtres UV (ou filtres solaires), les parfums, les ajusteurs de pH (par exemple acide citrique ou hydroxyde de sodium), et leurs mélanges.

13. Produit de soin de la peau, **caractérisé en ce qu'**il comprend une composition telle que définie à l'une quelconque des revendications 1 à 10.

14. Produit selon la revendication 11, **caractérisé en ce qu'**il constitue un sérum anti-âge, de préférence présentant un effet exfoliant, ou un démaquillant pour le visage, en particulier les yeux et/ou les lèvres.

15. Procédé de soin de la peau, consistant à appliquer sur la peau une composition telle que définie à l'une quelconque des revendications 1 à 10 et/ou un produit tel que défini à l'une quelconque des revendications 12 ou 13.

Phase huileuse
polarité faible

Phase huileuse
Polarité forte

**Biphase huileux**

Biphase huileux

Phase aqueuse

Triphase

Figure 1

**EP 3 895 683 A1**

| Europäisches Patentamt<br>European Patent Office<br>Office européen des brevets | **RAPPORT DE RECHERCHE EUROPEENNE** | Numéro de la demande<br>EP 20 31 5162 |
|---|---|---|

## DOCUMENTS CONSIDERES COMME PERTINENTS

| Catégorie | Citation du document avec indication, en cas de besoin,<br>des parties pertinentes | Revendication<br>concernée | CLASSEMENT DE LA<br>DEMANDE (IPC) |
|---|---|---|---|
| X | WO 2017/197587 A1 (ELCA COSMETICS LTD [CN]) 23 novembre 2017 (2017-11-23)<br>* revendications 1-15; exemples 1, 2 *<br>----- | 1-15 | INV.<br>A61K8/03<br>A61K8/31<br>A61K8/92<br>A61Q1/14<br>A61Q19/08 |
| X | EP 1 064 926 A1 (OREAL [FR]) 3 janvier 2001 (2001-01-03)<br>* revendications 1-8, 9-13; exemples 2-5 *<br>----- | 1-15 | |
| A | FR 2 939 662 A1 (OREAL [FR]) 18 juin 2010 (2010-06-18)<br>* le document en entier *<br>----- | 1-15 | |

DOMAINES TECHNIQUES
RECHERCHES (IPC)

A61K
A61Q

Le présent rapport a été établi pour toutes les revendications

| Lieu de la recherche | Date d'achèvement de la recherche | Examinateur |
|---|---|---|
| Munich | 2 octobre 2020 | Szarek, Sophie |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
...................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 20 31 5162

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-10-2020

| Document brevet cité au rapport de recherche | Date de publication | Membre(s) de la famille de brevet(s) | Date de publication |
|---|---|---|---|
| WO 2017197587 A1 | 23-11-2017 | AUCUN | |
| EP 1064926 A1 | 03-01-2001 | EP 1064926 A1<br>FR 2795632 A1<br>JP 2001031522 A | 03-01-2001<br>05-01-2001<br>06-02-2001 |
| FR 2939662 A1 | 18-06-2010 | FR 2939662 A1<br>WO 2010069995 A1 | 18-06-2010<br>24-06-2010 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3049459 A
- WO 2006134282 A
- WO 2009010356 A

**Littérature non-brevet citée dans la description**

- **STEFANIS, E. ; PANAYIOTOU, C.** Prediction of Hansen Solubility Parameters with a New Group-Contribution Method. *Int J Thermophys,* 2008, vol. 29, 568-585